# EUROPÄISCHE PATENTSCHRIFT

(11) **EP 3 885 335 B1**
(45) Veröffentlichungstag und Bekanntmachung des Hinweises auf die Patenterteilung: **03.05.2023**
(21) Anmeldenummer: 20020139.0
(22) Anmeldetag: 26.03.2020
(51) Int. Cl.: C07C 29/151, C07C 31/04, C07C 29/80, C07C 29/86

(54) **VERFAHREN UND ANLAGE ZUM HERSTELLEN VON REINMETHANOL**
METHOD AND SYSTEM FOR PRODUCING PURE METHANOL
PROCÉDÉ ET INSTALLATION DE FABRICATION DE MÉTHANOL PUR

(43) Veröffentlichungstag der Anmeldung: 29.09.2021
(73) Patentinhaber: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Gronemann, Veronika, 60439 Frankfurt (DE); Huder, Karin, 60439 Frankfurt (DE); Lim, Chin Han, 60439 Frankfurt (DE)
(74) Vertreter: Dropsch, Holger

(56) Entgegenhaltungen:
- EP-B1- 3 181 541
- WO-A1-2018/019875
- DE-A1- 3 220 995
- Chapter 5.2: "Synthesis" In: Orr et al.: "Ullmann's Encyclopedia of Industrial Chemistry", 2012, Wiley-VCH Verlag GmbH & Co. KGaA, Weinheim, Germany DOI: 10.1002/14356007.a16_465.pub3,
- ZHANG J ET AL: "A novel multi-effect methanol distillation process", CHEMICAL ENGINEERING AND PROCESSING: PROCESS INTENSIFICATION, ELSEVIER SEQUOIA, LAUSANNE, CH, vol. 49, no. 10, 1 October 2010 (2010-10-01), pages 1031-1037, XP027431429, ISSN: 0255-2701, DOI: 10.1016/J.CEP.2010.07.003 [retrieved on 2010-08-12]

## Beschreibung

### Gebiet der Erfindung

Die Erfindung betrifft ein Verfahren und eine Anlage zum Herstellen von Reinmethanol, bei dem der aus einer Methanol-Syntheseeinheit ausgeleitete Rohmethanolstrom in einem Entspannungsbehälter entspannt wird, danach in einer Vorlaufkolonne mindestens teilweise von leichtsiedenden Nebenprodukten befreit wird und sodann einer ein- oder mehrstufige Methanolreinigungsvorrichtung zugeführt wird, aus der schließlich ein Reinmethanolproduktstrom ausgeleitet wird.

### Stand der Technik

Methanol (MeOH) ist eine bedeutende organische Grundchemikalie und dient in der chemischen Industrie unter anderem als Ausgangsstoff für die Produktion von Formaldehyd, Ameisensäure und Essigsäure sowie auch als Ausgangsprodukt zur Herstellung von Olefinen. Die technische Methanolherstellung erfolgt hauptsächlich heterogen-katalytisch aus Kohlenoxiden und Wasserstoff.

Verfahren zur Herstellung von Methanol durch katalytische Umsetzung von Wasserstoff und Kohlenstoffoxide enthaltendem Synthesegas sind der Fachwelt seit langer Zeit bekannt. So werden in Ullmann's Encyclopedia of Industrial Chemistry, Sixth Edition, 1998 Electronic Release, Kapitel "Methanol", Unterkapitel 5.2 "Synthesis" verschiedene Grundverfahren zur Herstellung von Methanol beschrieben.

Ein spezifisches Verfahren zur Herstellung von Methanol ist beispielsweise aus der EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem Kupferkatalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280 °C vorgewärmt wird, bevor es in den ersten Synthesereaktor eingebracht wird. Ein Teil des zurückzuführenden Synthesegases wird als Spülstrom (sog. Purge) aus dem Verfahren entfernt, der gegenüber dem im System vorhandenen Gasinventar klein ist, um zu verhindern, dass sich Inertkomponenten, Verunreinigungen oder Nebenprodukte innerhalb des Synthesegaskreislaufes anreichern. Diese Maßnahme wird auch in der deutschen Offenlegungsschrift DE 2934332 A1 und der europäischen Patentanmeldung EP 1016643 A1 gelehrt.

Rohmethanol aus der Methanolsynthese enthält noch Wasser, höhere Alkohole, weitere Verunreinigungen und andere leichte Bestandteile. Um reines Methanol gemäß den auf dem Markt erhältlichen Spezifikationen herzustellen, muss das Rohmethanol durch Destillation gereinigt werden.

Wie beispielsweise im Fachbuch "Methanol: The Basic Chemical and Energy Feedstock of the Future: Asinger's Vision Today", Springer-Verlag, Berlin (2014), auf Seite 264 - 265 beschrieben wird, werden bei der destillativen Aufarbeitung von Rohmethanol zuerst gelöste Gase, beispielsweise Kohlenoxide, Wasserstoff und Methan, ausgetrieben, indem das Rohmethanol durch Einleiten in einen Expansionsbehälter entspannt wird. Die Entfernung der Leichtsieder, beispielsweise Ether, Formiate, Aldehyde, Ketone, und der restlichen gelösten Gase erfolgt in einer Vorlaufkolonne (Prerun-Kolonne). Anschließend wird das Methanol von den schweren Bestandteilen wie Ethanol, höhere Alkohole und Wasser in einer aus einer oder zwei Kolonnen bestehenden Reinmethanol-Destillationsanlage abgetrennt. Das Zweikolonnenverfahren mit einer Vorlaufkolonne und einer Reinmethanolkolonne weist dabei Vorteile hinsichtlich der Einsparung von Investitionskosten auf, während das Dreikolonnenverfahren, das neben der Vorlaufkolonne eine Druckkolonne und eine bei Atmosphärendruck betriebene Kolonne zur Reinmethanolgewinnung umfasst, Vorteile hinsichtlich des Energieverbrauchs bietet und sich besonders für große Produktionskapazitäten eignet.

Im Abgas der Prerun-Kolonne befindet sich noch etwas Methanol, das nicht kondensiert werden kann, weil sonst die mittels der Prerun-Kolonne entfernten Leichtsieder ebenfalls kondensieren und sich dort anreichern würden. Nachteilig bei den bislang aus dem Stand der Technik bekannten Verfahren zur destillativen Reinigung von Rohmethanol ist es daher, dass es zu Methanolverlusten kommt, wenn das Abgas über ein Fackelsystem thermisch entsorgt wird, oder dass Methanol in die Atmosphäre gelangt, wenn kein Fackelsystem zur Verfügung steht.

Darüber hinaus ist in den Destillationsanlagen ein oder mehrere Vorratsbehälter vorgesehen, in denen das Methanol im Falle einer Wartung zwischengelagert werden kann. Diese Vorratsbehälter müssen mit einem Inertgas, häufig mit Stickstoff gespült werden, damit sich kein Methanol in der Dampfphase ansammelt. Auch hier wird das methanolbeladene Inertgas bzw. Spülgas als Abgas abgeleitet und entsorgt, so dass wiederum Methanolverluste auftreten.

Die Patentpublikation DE 3220995 A1 beschreibt ein Verfahren zur Gewinnung von Methanol, bei dem aus einem kohlenstoffhaltigen Einsatzmaterial ein im wesentlichen Wasserstoff und Oxide des Kohlenstoffs enthaltendes Synthesegas erzeugt und unter Methanolsynthesebedingungen zur Reaktion gebracht wird, wobei ferner aus dem Reaktionsprodukt Rohmethanol abgetrennt und durch mehrstufige Destillation zu gereinigtem Methanol verarbeitet wird, wobei bei der Destillation anfallendes Kondensat mindestens teilweise vor die Methanolsynthese zurückgeführt wird.

Die Patentpublikation WO 2018/019875 A1 offenbart ein Verfahren zur Herstellung von Methanol durch Umsetzung von Kohlendioxid mit Wasserstoff, bei dem ein bei der Methanolsynthesereaktion erhaltener Produktstrom einem Hochdruckabscheider und/oder einem Niederdruckabscheider (28) zugeführt wird, in dem ein Gasstrom von einem Methanol-haltigen Produktstrom abgetrennt wird, wobei der Methanol-haltige Produktstrom anschließend mindestens einem Destillationsschritt (30) zugeführt wird, in dem mindestens eine Komponente, insbesondere Wasser, von dem Methanol-haltigen Produktstrom abgetrennt wird.

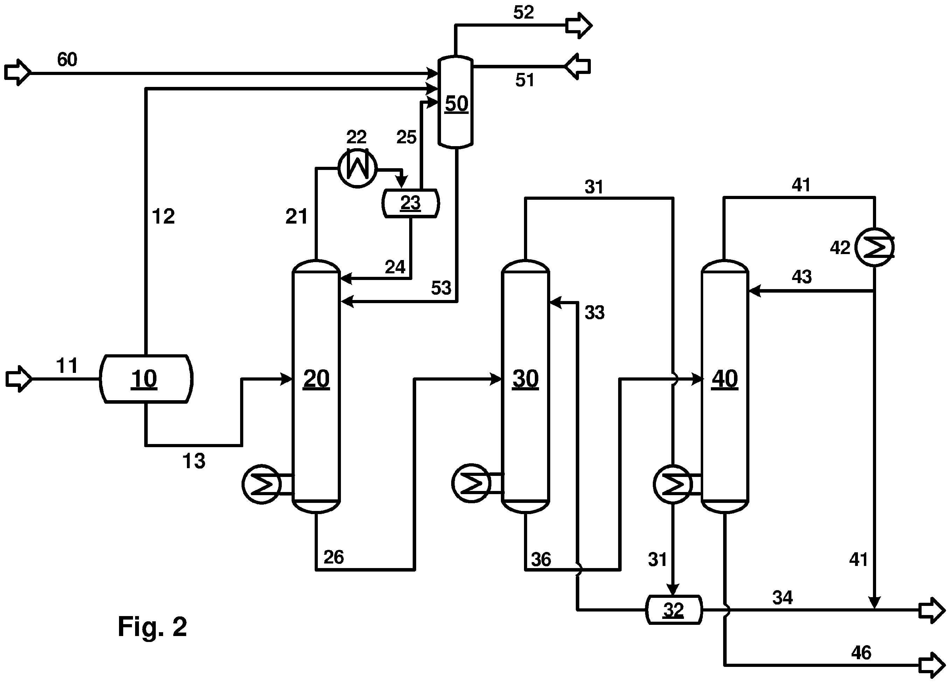

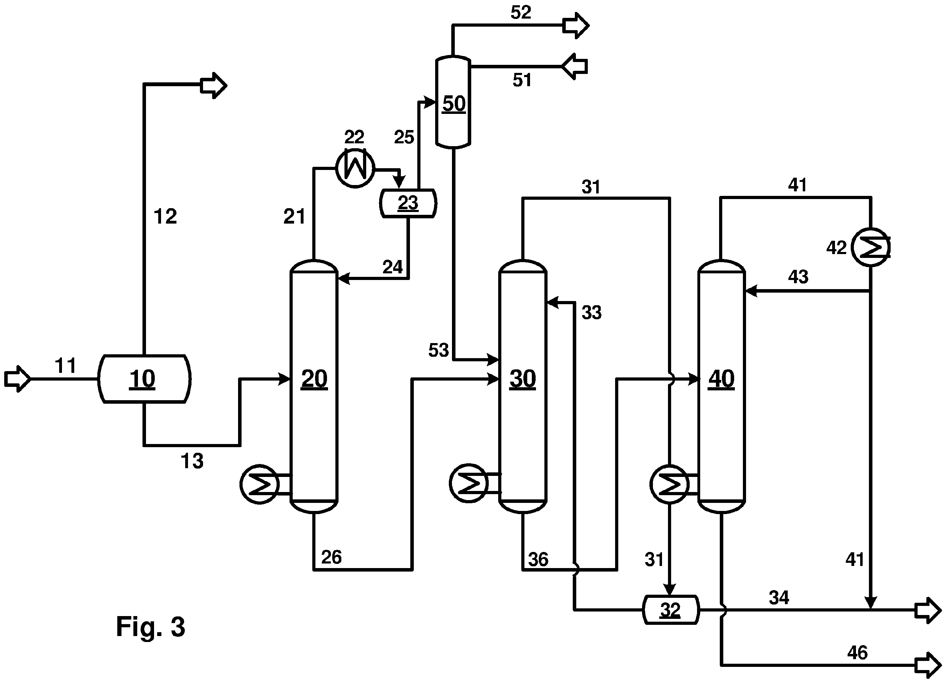

In der Veröffentlichung von Juntao Zhang et al., Chemical Engineering and Processing 49 (2010), Seiten 1031 bis 1037, wird die Reinigung vom Rohmethanol mittels einer Sequenz mehrerer Destillationskolonnen beschrieben. Ihnen vorgeschaltet ist eine Vorlaufkolonne ("pre-run column", vgl. dortige Fig. 2 und 3). Das aus dieser ausgeleitete, gasförmige Kopfprodukt wird mittels Wasserwäsche gewaschen. Das dabei erhaltene, mit Methanol beladene Waschwasser wird in die Vorlaufkolonne zurückgeleitet.

### Beschreibung der Erfindung

Die Aufgabe der vorliegenden Erfindung besteht daher darin, ein Verfahren und eine Anlage zum Herstellen von Reinmethanol anzugeben, die die erwähnten Nachteile des Stands der Technik nicht aufweisen.

Diese Aufgabe wird in einem ersten Aspekt durch ein Verfahren mit den Merkmalen des Anspruchs 1 und mit einer Anlage mit den Merkmalen des Anspruchs 5 gelöst. Weitere Ausgestaltungen der Erfindung ergeben sich aus den Unteransprüchen der jeweiligen Kategorie.

Die Methanolsynthesebedingungen sind dem Fachmann aus dem Stand der Technik, beispielsweise den eingangs erörterten Dokumenten, bekannt. Es sind diejenigen physikalisch-chemischen Bedingungen, unter denen ein messbarer, bevorzugt ein technisch relevanter Umsatz von Synthesegasbestandteilen zu Methanol erzielt wird. Notwendige Anpassungen dieser Bedingungen an die jeweiligen Betriebserfordernisse wird er auf der Grundlage von Routineversuchen vornehmen. Dabei können ggf. offenbarte, spezifische Reaktionsbedingungen als Orientierung dienen, sie sind aber in Bezug auf den Umfang der Erfindung nicht einschränkend zu verstehen.

Unter einem Aufteilen oder Auftrennen bzw. Abtrennen eines Stoffstroms ist im Zusammenhang mit der vorliegenden Erfindung die Erzeugung mindestens zweier Teilströme aus dem ursprünglichen Stoffstrom zu verstehen, wobei mit dem Auftrennen bzw. Abtrennen eine beabsichtigte Änderung der stofflichen Zusammensetzung der erhaltenen Teilströme in Bezug auf den ursprünglichen Stoffstrom verbunden ist, beispielsweise durch Anwendung eines thermischen Trennverfahrens auf den ursprünglichen Stoffstrom. Dagegen ist mit dem Aufteilen des ursprünglichen Stoffstroms in der Regel keine Änderung der stofflichen Zusammensetzung der erhaltenen Teilströme verbunden.

Unter einem Anreichern oder Abreichern einer Komponente in einem Gemisch, einer Fraktion oder einem Stoffstrom ist eine Maßnahme, Arbeitsschritt oder Verfahrensschritt zu verstehen, der dazu führt, dass sich der Stoffmengenanteil (Molenbruch) oder Massenanteil (Massenbruch) dieser Komponente erhöht (Anreichern) oder erniedrigt (Abreichern).

Unter dem überwiegenden Teil einer Fraktion, eines Stoffstroms etc. ist ein Anteil zu verstehen, der mengenmäßig größer ist als alle anderen jeweils für sich betrachteten Anteile. Insbesondere bei binären Gemischen oder bei der Auftrennung einer Fraktion in zwei Teile ist darunter ein Anteil von mehr als 50 Gew.-% zu verstehen, sofern im konkreten Fall nichts anderes angegeben ist.

Unter der Angabe, dass ein Stoffstrom überwiegend aus einer Komponente oder Komponentengruppe besteht, ist zu verstehen, dass der Stoffmengenanteil (Molenbruch) oder Massenanteil (Massenbruch) dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Massenanteil (Massenbruch) zugrunde gelegt.

Alle etwaigen Druckangaben erfolgen als Absolutdruckeinheiten, abgekürzt bar, oder als Überdruckeinheiten, abgekürzt barg, wenn im jeweiligen Einzelzusammenhang nichts anderes angegeben wird.

Unter Fluidverbindung zwischen zwei Bereichen der erfindungsgemäßen Vorrichtung wird dabei jegliche Art von Verbindung verstanden, die es ermöglicht, dass ein Fluid, beispielsweise ein Gasstrom, von dem einen zu dem anderen der beiden Bereiche strömen kann, unbeachtlich etwaiger zwischengeschalteter Bereiche oder Bauteile. Insbesondere wird unter einer direkten Fluidverbindung jegliche Art von Verbindung verstanden, die es ermöglicht, dass ein Fluid, beispielsweise ein Gasstrom, direkt von dem einen zu dem anderen der beiden Bereiche strömen kann, wobei keine weiteren Bereiche oder Bauteile zwischengeschaltet sind. Ein Beispiel wäre eine Rohrleitung, die direkt dem einen zu dem anderen der beiden Bereiche führt.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile, in Betracht ziehen, die ihm aufgrund seines Fachwissens zur Durchführung dieses Verfahrensschrittes notwendig oder sinnvoll erscheinen.

Unter der Angabe, dass zwei oder mehrere Verdichterstufen parallel geschaltet oder angeordnet sind, ist zu verstehen, dass ein Stoffstrom, der eine der Verdichterstufen durchläuft und dort verdichtet wird, nicht zusätzlich eine der anderen Verdichterstufen durchläuft und dort verdichtet wird, wobei die zwei oder mehreren Verdichterstufen unabhängig voneinander betrieben werden können.

Unter einer Abgasentsorgungsvorrichtung wird eine Vorrichtung verstanden, die nach einem Prinzip der Abgasnachbehandlung oder Abgasreinigung arbeitet. Die Abgasentsorgungsvorrichtung umfasst auch Mittel zum Abführen der Abgase von ihrem Entstehungsort und Zuführen zu der Abgasnachbehandlung oder Abgasreinigung, beispielsweise Rohrleitungen. Für brennbare Abgase ist oft das Verbrennen, beispielsweise in einem Fackelsystem, eine geeignete Entsorgungsmethode. Für weniger bedenkliche Abgasarten kann auch die Abgabe an die Umgebung, beispielsweise über einen Kamin, eine geeignete Entsorgungsmethode sein, wenn dabei die gesetzlichen Emissionsgrenzwerte eingehalten werden.

Unter einer Methanolreinigungsvorrichtung wird eine Vorrichtung verstanden, mittels derer Methanol in einem mehrstufigen Destillationsverfahren zu einem Reinmethanolprodukt aufgearbeitet werden kann. Hierzu zählen insbesondere die eingangs erörterten Zweikolonnenverfahren mit einer Vorlaufkolonne und einer Reinmethanolkolonne bzw. die Dreikolonnenverfahren, die neben der Vorlaufkolonne eine Druckkolonne und eine bei Atmosphärendruck betriebene Kolonne zur Reinmethanolgewinnung umfassen.

Gegenstand der vorliegenden Erfindung ist die Maximierung der Methanolausbeute. Dies geschieht neben anderen Maßnahmen in einem ersten Aspekt der Erfindung durch das Vorsehen einer Abgaswäsche, die die Leichtsieder nicht entfernt und daher nicht anreichert, sondern das selektiv Methanol zurückgewinnt, das in einer Ausgestaltung der Erfindung vom Boden der Waschvorrichtung zum Einlass der Vorlaufkolonne zurückgeführt wird.

### Bevorzugte Ausgestaltungen der Erfindung

Ein zweiter Aspekt des erfindungsgemäßen Verfahrens ist dadurch gekennzeichnet, dass als Waschmittel Wasser verwendet wird. Wasser als Waschmittel ist zumeist günstig, umweltneutral und in unterschiedlichen Reinheiten verfügbar. Mit Methanol beladenes, wässriges Waschmittel kann leicht in die Aufarbeitung des Rohmethanols zurückgeführt werden, da es mit Methanol und Wasser verfahrenseigenen Komponenten umfasst.

Ein weiterer Aspekt des erfindungsgemäßen Verfahrens ist dadurch gekennzeichnet, dass der gasförmige, Synthesegasbestandteile umfassende Kopfproduktstrom aus dem Entspannungsbehälter einem Waschschritt mit einem methanolselektiven Waschmittel in einer Waschvorrichtung unterzogen wird. Auch der Kopfproduktstrom des Entspannungsbehälters enthält noch Methanolanteile, die auf diese Weise sinnvoll wiedergewonnen werden können. Die Kombination der Methanol-Wiedergewinnung aus dem Kopfprodukt der Vorlaufkolonne mit derjenigen aus dem Kopfproduktstrom des Entspannungsbehälters erhöht den Anteil des insgesamt wiedergewonnen Methanols.

Ein weiterer Aspekt des erfindungsgemäßen Verfahrens ist dadurch gekennzeichnet, die Methanolreinigungsvorrichtung mindestens einen Methanol-Vorratsbehälter umfasst, dessen freier Gasraum mit einem Inertgas gespült wird, wobei das mit Methanol beladene Inertgas aus dem mindestens einen Methanol-Vorratsbehälter ausgeleitet und einem Waschschritt mit einem methanolselektiven Waschmittel in einer Waschvorrichtung unterzogen wird. Eine solche Methanolabtrennung aus dem Inertgas-Spülgas von Methanol-Vorratsbehältern wird beispielsweise in den europäischen Patentschriften EP 3181540 B1 und EP 3181541 B1 beschrieben. Die Kombination der Methanol-Wiedergewinnung aus dem Kopfprodukt der Vorlaufkolonne mit derjenigen aus dem Inertgas-Spülgas des oder der Methanol-Vorratsbehälter erhöht den Anteil des insgesamt wiedergewonnen Methanols. In Weiterbildung dieses Gedankens kann die Methanol-Wiedergewinnung dadurch maximiert werden, dass die Methanol-Wiedergewinnung aus dem Kopfprodukt der Vorlaufkolonne mit derjenigen aus dem Inertgas-Spülgas des oder der Methanol-Vorratsbehälter und derjenigen aus dem Kopfproduktstrom des Entspannungsbehälters kombiniert wird.

Ein weiterer Aspekt des erfindungsgemäßen Verfahrens ist dadurch gekennzeichnet, dass mindestens zwei der Waschschritte mit demselben Waschmittel in einer gemeinsamen Waschvorrichtung durchgeführt werden. Auf diese Weise kann die Methanol-Wiedergewinnung erhöht oder maximiert werden und es können aufgrund der gemeinsamen Nutzung Vorrichtungen und Ausrüstungsteile eingespart werden.

In einem weiteren Aspekt umfasst die erfindungsgemäße Anlage ferner Mittel zum Zuführen des gasförmigen, Synthesegasbestandteile umfassende Kopfproduktstroms aus dem Entspannungsbehälter zu der mindestens einen Waschvorrichtung und Mittel zum Ausleiten eines an Methanol abgereicherten Gasstroms aus der mindestens einen Waschvorrichtung. Die Vorteile dieses Aspektes der erfindungsgemäßen Anlage entsprechen denjenigen, die im Zusammenhang mit dem fünften Aspekt des erfindungsgemäßen Verfahrens erörtert wurden.

In einem weiteren Aspekt ist die erfindungsgemäße Anlage dadurch gekennzeichnet, dass die Methanolreinigungsvorrichtung mindestens einen Methanol-Vorratsbehälter umfasst, dessen freier Gasraum mit einem Inertgas spülbar ist, wobei ferner Mittel zum Ausleiten des mit Methanol beladenen Inertgases aus dem Methanol-Vorratsbehälter und zum Zuführen desselben zu der mindestens einen Waschvorrichtung umfasst werden. Die Vorteile dieses Aspektes der erfindungsgemäßen Anlage entsprechen denjenigen, die im Zusammenhang mit dem sechsten Aspekt des erfindungsgemäßen Verfahrens erörtert wurden.

In einem weiteren Aspekt ist die erfindungsgemäße Anlage dadurch gekennzeichnet, dass die mindestens eine Waschvorrichtung so ausgestaltet ist, dass mindestens zwei mit Methanol beladene Gasströme, ausgewählt aus der folgenden Gruppe, parallel in ihr gewaschen werden können: Kopfproduktstrom der Vorlaufkolonne, Kopfproduktstroms des Entspannungsbehälters, Inertgas aus dem Methanol-Vorratsbehälter. Die Vorteile dieses Aspektes der erfindungsgemäßen Anlage entsprechen denjenigen, die im Zusammenhang mit dem siebten Aspekt des erfindungsgemäßen Verfahrens erörtert wurden.

### Ausführungsbeispiele

Weiterbildungen, Vorteile und Anwendungsmöglichkeiten der Erfindung ergeben sich auch aus der nachfolgenden Beschreibung von Ausführungs- und Zahlenbeispielen und der Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Es zeigen:
Fig. 1 ein erstes Vergleichsbeispiel eines Verfahrens bzw. einer Anlage zum Herstellen von Reinmethanol gemäß des Stands der Technik,
Fig. 2 ein zweites Vergleichsbeispiel eines Verfahrens bzw. einer Anlage zum Herstellen von Reinmethanol gemäß der des Stands der Technik,
Fig. 3 ein Beispiel eines Verfahrens bzw. einer Anlage zum Herstellen von Reinmethanol gemäß der Erfindung.

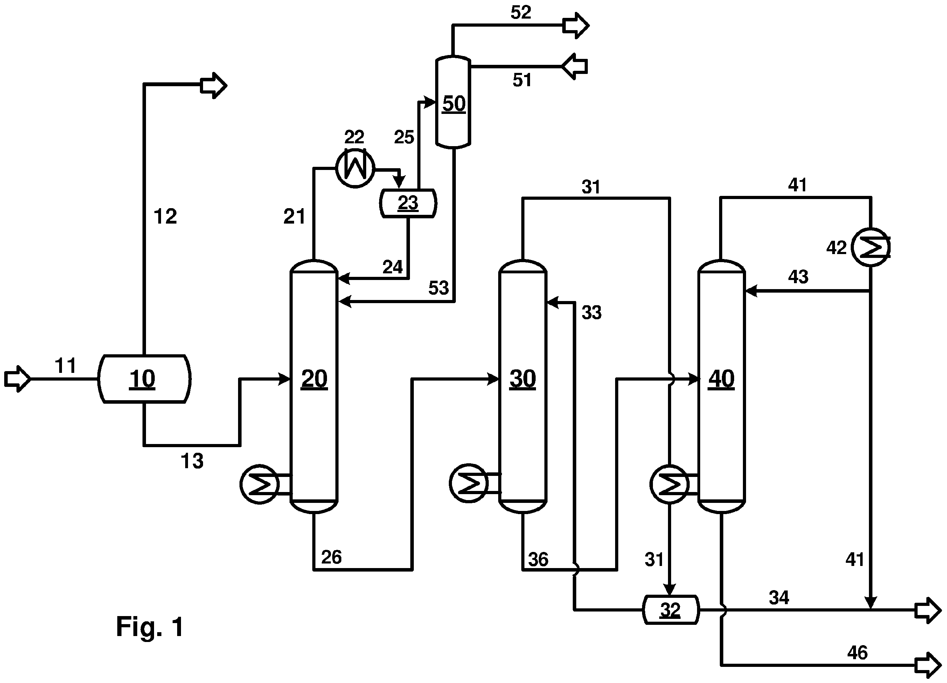

In dem in Fig. 1 gezeigten Vergleichsbeispiel gemäß Stand der Technik wird über Leitung 11 Rohmethanol, das durch mindestens teilweises Umsetzen eines Wasserstoff und Kohlenstoffoxide enthaltenden Synthesegases unter Methanolsynthesebedingungen in einer mindestens einen Methanol-Synthesereaktor enthaltenden, nicht bildlich gezeigten Methanol-Syntheseeinheit erzeugt wurde, in einen Entspannungsbehälter 10 geleitet und dort von einem Anfangsdruck von typischerweise 75 bara auf einen Enddruck von typischerweise 6 bara entspannt. Dabei werden in dem Rohmethanol gelöste Gase freigesetzt und über Leitung 12 als Kopfproduktstrom des Entspannungsbehälters einer nicht bildlich gezeigten Abgasentsorgung zugeführt.

Über Leitung 13 wird ein flüssiger, entspannter, an Synthesegasbestandteilen abgereicherter Rohmethanolstrom als Sumpfproduktstrom aus dem in Entspannungsbehälter ausgeleitet und in eine Vorlaufkolonne (Prerun-Kolonne) 20 eingeleitet. In dieser erfolgt eine destillative Auftrennung des an Synthesegasbestandteilen abgereicherten Rohmethanolstroms unter dem Fachmann bekannten Bedingungen. Über Leitung 21 wird ein gasförmiger, leichtsiedende Nebenprodukte und Methanoldampf umfassender Kopfproduktstrom aus der Vorlaufkolonne ausgeleitet. Nach Abkühlen desselben in einem Kühler 22 und Teilkondensation erfolgt eine Phasentrennung in einem Gas-Flüssig-Phasenabscheider 23. Aus diesem wird über Leitung 24 ein flüssiger Kondensatstrom zu der Vorlaufkolonne zurückgeführt und über Leitung 25 ein Gasstrom ausgeleitet. Erfindungsgemäß wird dieser Gasstrom über Leitung 25 in eine Waschvorrichtung 50 eingeleitet und dort mit Wasser als methanolselektivem Waschmittel gewaschen, das über Leitung 51 herangeführt und in die Waschvorrichtung in deren oberen Bereich, vorzugsweise an deren Kopfende eingeleitet wird. Die technische Durchführung dieses Waschverfahrens ist dem Fachmann an sich bekannt. Detailinformationen kann er ferner dem einschlägigen Schrifttum entnehmen, beispielsweise den beiden bereits erwähnten EP-Patentschriften EP 3181540 B1 und EP 3181541 B1.

Das mit Methanol beladene Waschmittel wird in Fig. 1 über 53 zu der Vorlaufkolonne zurückgeführt und in diese eingeleitet. Über Leitung 52 wird der von Methanol befreite, leichtsiedende Nebenprodukte der Methanolsynthese enthaltende Gasstrom aus der Waschvorrichtung ausgeleitet und der Abgasentsorgung zugeführt. Diese umfasst vorzugsweise ein Fackelsystem, so dass auch brennbare, leichtsiedende Nebenprodukte der Methanolsynthese thermisch abgebaut und somit umweltneutral an die Umgebung abgegeben werden können.

Über Leitung 26 wird ein flüssiger, stabilisierter, an leichtsiedenden Nebenprodukten abgereicherter Methanolstrom als Sumpfproduktstrom aus der Vorlaufkolonne ausgeleitet und in eine Druckkolonne 30 eingeleitet, die bei einem Druck von typischerweise 8,6 bara betrieben wird. In dieser erfolgt die destillative Abtrennung von Reinmethanol als dampfförmiges Kopfprodukt, das über Leitung 31 ausgeleitet wird. Im Leitungsweg 31 befindet sich ein Wärmetauscher, der der Beheizung einer nachgeschalteten, atmosphärischen Kolonne 40 dient, wobei als Heizmedium der aus der Druckkolonne ausgeleitete Reinmethanoldampf verwendet und dabei gleichzeitig abgekühlt und teilkondensiert wird. Die dabei erfolgte Phasentrennung und die Führung der Dampfphase wurde aus Vereinfachungsgründen nicht bildlich dargestellt. Über Leitung 31, Behälter 32 und Leitung 34 wird ein flüssiger Reinmethanolstrom als Zielprodukt aus dem Verfahren bzw. der Anlage ausgeleitet. Über Leitung 33 wird ein Reinmethanol-Teilstrom zu der Druckkolonne zurückgeführt.

Der Methanol und Wasser enthaltende Sumpfproduktstrom der Druckkolonne wird über Leitung 36 aus dieser ausgeleitet und in die atmosphärische Kolonne 40 eingeleitet. In dieser erfolgt die weitere, destillative Auftrennung des Methanol-Wasser-Gemisches bei Atmosphärendruck und unter dem Fachmann bekannten sonstigen Bedingungen. Das dabei erhaltene, überwiegend Wasser enthaltende Sumpfprodukt wird über Leitung 46 aus der Kolonne 40 ausgeleitet und einer bildlich nicht dargestellten Abwasserentsorgung zugeführt.

Über Leitung 41 wird ein Reinmethanol-Dampfstrom aus der Kolonne 40 ausgeleitet und nachfolgend im Kühler 42 abgekühlt und dabei teilkondensiert. Die dabei erfolgte Phasentrennung und die Führung der Dampfphase wurde aus Vereinfachungsgründen nicht bildlich dargestellt. Ein Teilstrom des Reinmethanol-Kondensats wird als Rücklaufstrom über Leitung 43 zu der Kolonne 40 zurückgeführt und dieser in deren oberen Bereich aufgegeben. Das verbleibende Reinmethanol-Kondensat wird über Leitungen 41 und 34 dem flüssigen Reinmethanolstrom zugegeben.

In dem in Fig. 2 gezeigten Vergleichsbeispiel gemäß Stand der Technik wird zusätzlich zu Fig. 1 auch der Kopfproduktstrom des Entspannungsbehälters, der die im Rohmethanol gelösten Gase enthält, über Leitung 12 ausgeleitet und zu der Waschvorrichtung 50 geführt und in diese eingeleitet. Ferner wird über Leitung 60 ein mit Methanoldampf beladener Spülgasstrom, der zuvor aus einem bildlich nicht dargestellten Methanol-Vorratsbehälter ausgeleitet wurde, zu der Waschvorrichtung 50 geführt und in diese eingeleitet. Vorteilhaft ist es dabei, dass die Waschvorrichtung auch zur Entfernung von Methanol aus den in den Leitungen 12 und 60 geführten Gasströmen verwendet werden kann, ohne dass zusätzliche, separate Waschvorrichtungen vorgesehen werden müssen. Es kann auch entweder nur der Gasstrom in Leitung 12 oder nur der Gasstrom in Leitung 60 zu der Waschvorrichtung 50 geführt werden.

In dem in Fig. 3 gezeigten Ausführungsbeispiel der Erfindung wird in Abänderung der Fig. 1 der über Leitung 53 aus der Waschvorrichtung 50 ausgeleitete, mit Methanol beladene Waschmittel enthaltende Sumpfproduktstrom zu der Druckkolonne 30 geleitet und in diese eingeführt. Auch dieses Ausführungsbeispiel der Erfindung kann so abgewandelt werden, dass wie im Vergleichsbeispiel der Fig. 2 auch der Gasstrom in Leitung 12 und/oder ein mit Methanoldampf beladener Spülgasstrom aus einem Methanol-Vorratsbehälter (Fig. 2, Leitung 60) zu der Waschvorrichtung 50 geführt werden. Eine weitere Option besteht darin, jeweils einen Teilstrom des mit Methanol beladenen Waschmittels aus der Waschvorrichtung 50 der Vorlaufkolonne und der Druckkolonne zuzuführen. Auf diese Weise kann das Gesamtverfahren empfindlicher gesteuert werden, beispielsweise im Falle von Inbetriebnahmen oder Verfahrensumstellungen.

### Zahlenbeispiel

Das erfindungsgemäße Verfahren wurde gemäß der in Fig. 3 gezeigten Ausgestaltung durchgeführt. Der über Leitung 25 aus der Vorlaufkolonne ausgeleitete Gasstrom wurde in die Waschvorrichtung 50 eingeleitet, die als Waschkolonne mit Füllkörpern oder Böden ausgestaltet war, und dort mit demineralisiertem Wasser gewaschen. Dabei wurden rund 90 Gew.-% des Methanols als Flüssigkeit zurückgewonnen, während alle noch in die Vorlaufkolonne gelangten, gelösten Gase und mehr als 80 Gew.-% der Leichtsieder die Waschvorrichtung über Kopf verließen und zur Abgasentsorgung gelangten.

### Bezugszeichenliste

- [10]: Entspannungsbehälter
- [11]: Leitung
- [12]: Leitung
- [13]: Leitung
- [20]: Vorlaufkolonne
- [21]: Leitung
- [22]: Kühler
- [23]: Gas-Flüssig-Phasenabscheider
- [24]: Leitung
- [25]: Leitung
- [26]: Leitung
- [30]: Druckkolonne
- [31]: Leitung
- [32]: Behälter
- [33]: Leitung
- [34]: Leitung
- [36]: Leitung
- [40]: atmosphärische Kolonne
- [41]: Leitung
- [42]: Kühler
- [43]: Leitung
- [46]: Leitung
- [50]: Waschvorrichtung
- [51]: Leitung
- [52]: Leitung
- [53]: Leitung
- [60]: Leitung

## Patentansprüche

1. Verfahren zum Herstellen von Reinmethanol aus einem Wasserstoff und Kohlenstoffoxide als Synthesegasbestandteile enthaltenden Synthesegas, umfassend folgende Schritte:
(a) Mindestens teilweises Umsetzen des Wasserstoff und Kohlenstoffoxide enthaltenden Synthesegases unter Methanolsynthesebedingungen in einer mindestens einen Methanol-Synthesereaktor enthaltenden Methanol-Syntheseeinheit,
(b) Ausleiten eines flüssigen Rohmethanolstroms, enthaltend Methanol, Wasser, gelöste Synthesegasbestandteile und leichtsiedende Nebenprodukte, aus der Methanol-Syntheseeinheit,
(c) Einleiten des flüssigen Rohmethanolstroms in einen Entspannungsbehälter, Ausleiten eines flüssigen, entspannten, an Synthesegasbestandteilen abgereicherten Rohmethanolstroms als Sumpfprodukt und eines gasförmigen, Synthesegasbestandteile umfassenden Kopfproduktstroms aus dem Entspannungsbehälter,
(d) Einleiten des flüssigen, entspannten, an Synthesegasbestandteilen abgereicherten Rohmethanolstroms in eine Vorlaufkolonne, destillative Auftrennung desselben, Ausleiten eines flüssigen, stabilisierten, an leichtsiedenden Nebenprodukten abgereicherten Methanolstroms als Sumpfproduktstrom und eines gasförmigen, leichtsiedende Nebenprodukte und Methanoldampf umfassenden Kopfproduktstroms aus der Vorlaufkolonne,
(e) Einleiten des Sumpfproduktstroms der Vorlaufkolonne in eine ein- oder mehrstufige Methanolreinigungsvorrichtung, Ausleiten eines Reinmethanolproduktstroms aus der Methanolreinigungsvorrichtung,
(f) Zuführen des Kopfproduktstroms der Vorlaufkolonne zu einer Abgasentsorgungsvorrichtung,
**dadurch gekennzeichnet, dass**
(g) der Kopfproduktstrom der Vorlaufkolonne vor dem Zuführen zu der Abgasentsorgungsvorrichtung einem Waschschritt mit einem methanolselektiven Waschmittel in einer Waschvorrichtung unterzogen und ein an Methanol abgereicherter Kopfproduktstrom der Abgasentsorgungsvorrichtung zugeführt wird, wobei als Waschmittel Wasser verwendet wird und wobei aus der Waschvorrichtung mit Methanol beladenes Wasser ausgeleitet und in die Methanolreinigungsvorrichtung eingeleitet wird.

2. Verfahren nach Anspruch 1, **dadurch gekennzeichnet, dass** der gasförmige, Synthesegasbestandteile umfassende Kopfproduktstrom aus dem Entspannungsbehälter einem Waschschritt mit einem methanolselektiven Waschmittel in einer Waschvorrichtung unterzogen wird.

3. Verfahren nach einem der vorigen Ansprüche, **dadurch gekennzeichnet, dass** die Methanolreinigungsvorrichtung mindestens einen Methanol-Vorratsbehälter umfasst, dessen freier Gasraum mit einem Inertgas gespült wird, wobei das mit Methanol beladene Inertgas aus dem mindestens einen Methanol-Vorratsbehälter ausgeleitet und einem Waschschritt mit einem methanolselektiven Waschmittel in einer Waschvorrichtung unterzogen wird.

4. Verfahren nach einem der vorigen Ansprüche, **dadurch gekennzeichnet, dass** mindestens zwei der Waschschritte mit demselben Waschmittel in einer gemeinsamen Waschvorrichtung durchgeführt werden.

5. Anlage zum Herstellen von Reinmethanol aus einem Wasserstoff und Kohlenstoffoxide als Synthesegasbestandteile enthaltenden Synthesegas, umfassend folgende, miteinander in Fluidverbindung stehende Baugruppen und Anlagenbestandteile:
(a) eine Methanol-Syntheseeinheit, umfassend mindestens einen Methanol-Synthesereaktor, und Mittel zum Zuführen des Wasserstoff und Kohlenstoffoxide als Synthesegasbestandteile enthaltenden Synthesegases zu der Methanol-Syntheseeinheit,
(b) Mittel zum Ausleiten eines flüssigen Rohmethanolstroms, enthaltend Methanol, Wasser, gelöste Synthesegasbestandteile und leichtsiedende Nebenprodukte, aus der Methanol-Syntheseeinheit,
(c) einen Entspannungsbehälter, Mittel zum Einleiten des flüssigen Rohmethanolstroms in den Entspannungsbehälter, Mittel zum Ausleiten eines flüssigen, entspannten, an Synthesegasbestandteilen abgereicherten Rohmethanolstroms als Sumpfprodukt und eines gasförmigen, Synthesegasbestandteile umfassenden Kopfproduktstroms aus dem Entspannungsbehälter,
(d) eine als Destillationskolonne ausgestaltete Vorlaufkolonne, Mittel zum Einleiten des flüssigen, entspannten, an Synthesegasbestandteilen abgereicherten Rohmethanolstroms in die Vorlaufkolonne, Mittel zum Ausleiten eines flüssigen, stabilisierten, an leichtsiedenden Nebenprodukten abgereicherten Methanolstroms als Sumpfproduktstrom und eines gasförmigen, leichtsiedende Nebenprodukte und Methanoldampf umfassenden Kopfproduktstroms aus der Vorlaufkolonne,
(e) eine ein- oder mehrstufige Methanolreinigungsvorrichtung, Mittel zum Einleiten des Sumpfproduktstroms der Vorlaufkolonne in die ein- oder mehrstufige Methanolreinigungsvorrichtung, Mittel zum Ausleiten eines Reinmethanolproduktstroms aus der Methanolreinigungsvorrichtung,
(f) eine Abgasentsorgungsvorrichtung, Mittel zum Zuführen des Kopfproduktstroms der Vorlaufkolonne zu der Abgasentsorgungsvorrichtung,
**dadurch gekennzeichnet, dass** die Anlage ferner umfasst:
(g) mindestens eine Waschvorrichtung, geeignet zum Abtrennen von Methanol aus Gasen mit einem methanolselektiven Waschmittel, Mittel zum Zuführen des Kopfproduktstroms der Vorlaufkolonne zu der Waschvorrichtung, Mittel zum Ausleiten eines an Methanol abgereicherten Gasstroms aus der Waschvorrichtung und Mittel zum Zuführen desselben zu der Abgasentsorgungsvorrichtung, ferner umfassend Mittel zum Ausleiten von mit Methanol beladenem Waschmittel aus der mindestens einen Waschvorrichtung und Mittel zum Zuführen desselben zu der Methanolreinigungsvorrichtung.

6. Anlage nach Anspruch 5, ferner umfassend Mittel zum Zuführen des gasförmigen, Synthesegasbestandteile umfassende Kopfproduktstroms aus dem Entspannungsbehälter zu der mindestens einen Waschvorrichtung und Mittel zum Ausleiten eines an Methanol abgereicherten Gasstroms aus der mindestens einen Waschvorrichtung.

7. Anlage nach einem der vorigen Ansprüche, **dadurch gekennzeichnet, dass** die Methanolreinigungsvorrichtung mindestens einen Methanol-Vorratsbehälter umfasst, dessen freier Gasraum mit einem Inertgas spülbar ist, wobei ferner Mittel zum Ausleiten des mit Methanol beladenen Inertgases aus dem Methanol-Vorratsbehälter und zum Zuführen desselben zu der mindestens einen Waschvorrichtung umfasst werden.

8. Anlage nach einem der vorigen Ansprüche, **dadurch gekennzeichnet, dass** die mindestens eine Waschvorrichtung so ausgestaltet ist, dass mindestens zwei mit Methanol beladene Gasströme, ausgewählt aus der folgenden Gruppe, parallel in ihr gewaschen werden können: Kopfproduktstrom der Vorlaufkolonne, Kopfproduktstroms des Entspannungsbehälters, Inertgas aus dem Methanol-Vorratsbehälter.

## Claims

1. Process for producing pure methanol from a synthesis gas containing hydrogen and carbon oxides as synthesis gas constituents, comprising the steps of:
(a) at least partially converting the synthesis gas containing hydrogen and carbon oxides under methanol synthesis conditions in a methanol synthesis unit containing at least one methanol synthesis reactor,
(b) discharging a liquid crude methanol stream containing methanol, water, dissolved synthesis gas constituents and low-boiling byproducts from the methanol synthesis unit,
(c) introducing the liquid crude methanol stream into a decompression vessel, discharging a liquid, decompressed crude methanol stream depleted in synthesis gas constituents as the bottoms product and a gaseous tops product stream comprising synthesis gas constituents from the decompression vessel,
(d) introducing the liquid, decompressed crude methanol stream depleted in synthesis gas constituents to a prerun column, distillatively resolving said stream, discharging a liquid, stabilized, methanol stream depleted in low-boiling byproducts as the bottoms product stream and a gaseous tops product stream comprising low-boiling byproducts and methanol vapour from the prerun column,
(e) introducing the bottoms product stream from the prerun column to a single- or multi-stage methanol purification apparatus, discharging a pure methanol product stream from the methanol purification apparatus,
(f) sending the tops product stream from the prerun column to an offgas disposal apparatus,
**characterized in that**
(g) before sending to the offgas disposal apparatus, the tops product stream from the prerun column is subjected to a scrubbing step with a methanol-selective scrubbing medium in a scrubbing apparatus and a methanol-depleted tops product stream is sent to the offgas disposal apparatus, wherein water is used as the scrubbing medium and wherein methanol-laden water is discharged from the scrubbing apparatus and introduced to the methanol purification apparatus.

2. Process according to Claim 1, **characterized in that** the gaseous tops product stream comprising synthesis gas constituents from the decompression vessel is subjected to a scrubbing step with a methanol-selective scrubbing medium in a scrubbing apparatus.

3. Process according to either of the preceding claims, **characterized in that** the methanol purification apparatus comprises at least one methanol reservoir vessel whose free gas space is purged with an inert gas, wherein the methanol-laden inert gas is discharged from the at least one methanol reservoir vessel and subjected to a scrubbing step with a methanol-selective scrubbing medium in a scrubbing apparatus.

4. Process according to any of the preceding claims, **characterized in that** at least two of the scrubbing steps are performed with the same scrubbing medium in a common scrubbing apparatus.

5. Plant for producing pure methanol from a synthesis gas containing hydrogen and carbon oxides as synthesis gas constituents, comprising the following assemblies and plant constituents in fluid connection with one another:
(a) a methanol synthesis unit comprising at least one methanol synthesis reactor and means for supplying the synthesis gas containing hydrogen and carbon oxides as synthesis gas constituents to the methanol synthesis unit,
(b) means for discharging a liquid crude methanol stream containing methanol, water, dissolved synthesis gas constituents and low-boiling byproducts from the methanol synthesis unit,
(c) a decompression vessel, means for introducing the liquid crude methanol stream to the decompression vessel, means for discharging a liquid, decompressed crude methanol stream depleted in synthesis gas constituents as the bottoms product and a gaseous tops product stream comprising synthesis gas constituents from the decompression vessel,
(d) a prerun column configured as a distillation column, means for introducing the liquid, decompressed crude methanol stream depleted in synthesis gas constituents to the prerun column, means for discharging a liquid, stabilized, methanol stream depleted in low-boiling byproducts as the bottoms product stream and a gaseous tops product stream comprising low-boiling byproducts and methanol vapour from the prerun column,
(e) a single- or multi-stage methanol purification apparatus, means for introducing the bottoms product stream from the prerun column into the single- or multi-stage methanol purification apparatus, means for discharging a pure methanol product stream from the methanol purification apparatus,
(f) an offgas disposal apparatus, means for sending the tops product stream from the prerun column to the offgas disposal apparatus,
**characterized in that** the plant further comprises:
(g) at least one scrubbing apparatus suitable for separating methanol from gases with a methanol-selective scrubbing medium, means for supplying the tops product stream from the prerun column to the scrubbing apparatus, means for discharging a methanol-depleted gas stream from the scrubbing apparatus and means for sending same to the offgas disposal apparatus, further comprising means for discharging methanol-laden scrubbing medium from the at least one scrubbing apparatus and means for supplying same to the methanol purification apparatus.

6. Plant according to Claim 5, further comprising means for supplying the gaseous tops product stream comprising synthesis gas constituents from the decompression vessel to the at least one scrubbing apparatus and means for discharging a methanol-depleted gas stream from the at least one scrubbing apparatus.

7. Plant according to any of the preceding claims, **characterized in that** the methanol purification apparatus comprises at least one methanol reservoir vessel whose free gas space is purgable with an inert gas, wherein the plant further comprises means for discharging the methanol-laden inert gas from the methanol reservoir vessel and for supplying same to the at least one scrubbing apparatus.

8. Plant according to any of the preceding claims, **characterized in that** the at least one scrubbing apparatus is configured such that at least two methanol-laden gas streams selected from the following group may be scrubbed therein simultaneously: tops product stream from the prerun column, tops product stream from the decompression vessel, inert gas from the methanol reservoir vessel.

## Revendications

1. Procédé de préparation de méthanol pur à partir d'un gaz de synthèse contenant de l'hydrogène et des oxydes de carbone en tant qu'ingrédients de gaz de synthèse, comprenant les étapes suivantes :
(a) transformation au moins partielle du gaz de synthèse contenant de l'hydrogène et des oxydes de carbone dans des conditions de synthèse de méthanol dans au moins un réacteur de synthèse de méthanol contenant une unité de synthèse de méthanol,
(b) évacuation d'un flux de méthanol brut liquide, contenant du méthanol, de l'eau, des ingrédients de gaz de synthèse dissous et des sous-produits volatils, de l'unité de synthèse de méthanol,
(c) introduction du flux de méthanol brut liquide dans un récipient de détente, évacuation d'un flux de méthanol brut liquide, détendu, appauvri en ingrédients de gaz de synthèse en tant que produit de fond et d'un flux gazeux de produit de tête comprenant des ingrédients de gaz de synthèse du récipient de détente,
(d) introduction du flux de méthanol brut liquide, détendu, appauvri en ingrédients de gaz de synthèse dans une colonne préliminaire, séparation par distillation de celui-ci, évacuation d'un flux de méthanol liquide, stabilisé, appauvri en sous-produits à bas point d'ébullition en tant que flux de produit de fond et d'un flux gazeux de produit de tête, comprenant des sous-produits à bas point d'ébullition et de la vapeur de méthanol de la colonne préliminaire,
(e) introduction du flux de produit de fond de la colonne préliminaire dans un dispositif de purification de méthanol à un étage ou à plusieurs étages, évacuation d'un flux de produit de méthanol pur du dispositif de purification de méthanol,
(f) alimentation du flux de produit de tête de la colonne préliminaire à, un dispositif d'élimination de gaz d'échappement,
**caractérisé en ce que**
(g) le flux de produit de tête de la colonne préliminaire, avant l'alimentation au dispositif d'élimination de gaz d'échappement, est soumis à une étape de lavage avec un agent de lavage sélectif pour le méthanol dans un dispositif de lavage et un flux de produit de tête appauvri en méthanol est alimenté au dispositif d'élimination de gaz d'échappement, de l'eau étant utilisée comme agent de lavage et de l'eau chargée avec du méthanol étant évacuée du dispositif de lavage et introduite dans le dispositif de purification de méthanol.

2. Procédé selon la revendication 1, **caractérisé en ce que** le flux de produit de tête gazeux comprenant des ingrédients de gaz de synthèse, provenant du récipient de détente est soumis à une étape de lavage avec un agent de lavage sélectif pour le méthanol dans un dispositif de lavage.

3. Procédé selon l'une quelconque des revendications précédentes, **caractérisé en ce que** le dispositif de purification de méthanol comprend au moins un récipient de réserve de méthanol, dont l'espace de gaz libre est purgé avec un gaz inerte, le gaz inerte chargé de méthanol étant évacué de l' au moins un récipient de réserve de méthanol et étant soumis à une étape de lavage avec un agent de lavage sélectif pour le méthanol dans un dispositif de lavage.

4. Procédé selon l'une quelconque des revendications précédentes, **caractérisé en ce qu'**au moins deux des étapes de lavage sont réalisées avec le même agent de lavage dans un dispositif de lavage commun.

5. Installation de préparation de méthanol pur à partir d'un gaz de synthèse contenant de l'hydrogène et des oxydes de carbone en tant qu'ingrédients de gaz de synthèse, comprenant les modules et les éléments d'installation suivants, qui se trouvent en liaison fluidique les uns avec les autres :
(a) une unité de synthèse de méthanol, comprenant au moins un réacteur de synthèse de méthanol, et un moyen pour l'alimentation du gaz de synthèse contenant de l'hydrogène et des oxydes de carbone en tant qu'ingrédients de gaz de synthèse à l'unité de synthèse de méthanol,
(b) un moyen d'évacuation d'un flux de méthanol brut liquide, contenant du méthanol, de l'eau, des ingrédients de gaz de synthèse dissous et des sous-produits à bas point d'ébullition, de l'unité de synthèse de méthanol,
(c) un récipient de détente, des moyens pour l'introduction du flux de méthanol brut liquide dans le récipient de détente, des moyens pour l'évacuation d'un flux de méthanol brut liquide, détendu, appauvri en ingrédients de gaz de synthèse en tant que produit de fond et d'un flux gazeux de produit de tête comprenant des ingrédients de gaz de synthèse du récipient de détente,
(d) une colonne préliminaire conçue comme colonne de distillation, des moyens pour l'introduction du flux de méthanol brut liquide, détendu, appauvri en ingrédients de gaz de synthèse dans la colonne préliminaire, des moyens pour l'évacuation d'un flux de méthanol liquide, stabilisé, appauvri en sous-produits à bas point d'ébullition en tant que flux de produit de fond et d'un flux gazeux de produit de tête, comprenant des sous-produits à bas point d'ébullition et de la vapeur de méthanol de la colonne préliminaire,
(e) un dispositif de purification de méthanol à un étage ou à plusieurs étages, des moyens pour l'introduction du flux de produit de fond de la colonne préliminaire dans le dispositif de purification de méthanol à un étage ou à plusieurs étages, des moyens pour l'évacuation d'un flux de produit de méthanol pur du dispositif de purification de méthanol,
(f) un dispositif d'élimination de gaz d'échappement, des moyens d'alimentation du flux de produit de tête de la colonne préliminaire au dispositif d'élimination de gaz d'd'échappement,
**caractérisée en ce que** l'installation comprend en outre :
(g) au moins un dispositif de lavage, approprié pour la séparation de méthanol de gaz avec un agent de lavage sélectif pour le méthanol, des moyens d'alimentation du flux de produit de tête de la colonne préliminaire au dispositif de lavage, des moyens pour l'évacuation d'un flux de gaz appauvri en méthanol du dispositif de lavage et des moyens d'alimentation de celui-ci au dispositif d'élimination de gaz d'échappement, comprenant en outre des moyens d'évacuation d'agent de lavage chargé en méthanol de l'au moins un dispositif de lavage et des moyens d'alimentation de celui-ci au dispositif de purification de méthanol,

6. Installation selon la revendication 5, comprenant en outre des moyens d'alimentation du flux gazeux de produit de tête comprenant des ingrédients de gaz de synthèse provenant du récipient de détente à l'au moins un dispositif de lavage et des moyens d'évacuation d'un flux de gaz appauvri en méthanol de l'au moins un dispositif de lavage.

7. Installation selon l'une quelconque des revendications précédentes, **caractérisée en ce que** le dispositif de purification de méthanol comprend au moins un récipient de réserve de méthanol, dont l'espace de gaz libre peut être purgé avec un gaz inerte, en outre des moyens d'évacuation du gaz inerte chargé en méthanol du récipient de réserve de méthanol et d'alimentation de celui-ci à l'au moins un dispositif de lavage étant compris.

8. Installation selon l'une quelconque des revendications précédentes, **caractérisée en ce que** l'au moins un dispositif de lavage est conçu de telle manière qu'au moins deux flux de gaz chargés en méthanol, choisis dans le groupe suivant, peuvent être lavés dans celui-ci en parallèle : flux de produit de tête de la colonne préliminaire, flux de produit de tête du récipient de détente, gaz inerte provenant du récipient de réserve de méthanol.
